# EUROPÄISCHE PATENTSCHRIFT

(11) **EP 3 127 601 B1**
(45) Veröffentlichungstag und Bekanntmachung des Hinweises auf die Patenterteilung: **06.07.2022**
(21) Anmeldenummer: 16180536.1
(22) Anmeldetag: 21.07.2016
(51) Int. Cl.: B01F 31/441, B01F 35/71, B01F 35/75, B01F 101/20

(54) **MISCHVORRICHTUNG, INSBESONDERE FÜR KNOCHENZEMENT**
MIXING DEVICE, IN PARTICULAR FOR BONE CEMENT
DISPOSITIF DE MELANGE, EN PARTICULIER POUR CIMENT OSSEUX

(30) Priorität: 27.07.2015 DE 102015112203
(43) Veröffentlichungstag der Anmeldung: 08.02.2017
(73) Patentinhaber: OSARTIS GmbH, 64839 Münster (DE)
(72) Erfinder: Sattig, Christoph, verstorben (DE); Reuschenbach, Ingeborg, 64839 Münster (DE)
(74) Vertreter: Blumbach · Zinngrebe Patentanwälte PartG mbB

(56) Entgegenhaltungen:
- EP-A1- 2 457 531
- WO-A2-2005/048867
- DE-A1-102007 026 034
- DE-A1-102012 024 710
- US-B1- 6 379 033

## Beschreibung

### Gebiet der Erfindung

Die Erfindung bezieht sich auf eine Mischvorrichtung, insbesondere eine Mischvorrichtung, wie sie zum Anmischen von Knochenzement verwendet wird.

### Hintergrund der Erfindung

Knochenzemente, insbesondere polymerbasierte Knochenzemente, bestehen in der Regel aus einer Pulverkomponente und einer Flüssigkomponente. Zum Anmischen des Knochenzements wird in der Regel eine Mischvorrichtung verwendet, die einen Mischraum umfasst, in welchem die Pulverkomponente und die Flüssigkomponente zu einer gut verarbeitbaren pastösen Masse zusammen gemischt werden.

Eine Mischvorrichtung für Knochenzement ist aus der DE 10 2007 026 034 A1 (aap Biomaterials GmbH & Co. KG) bekannt.

Diese Mischvorrichtung ist eine Mischvorrichtung, insbesondere für Knochenzement, umfassend ein Gehäuse mit einem Mischraum, wobei die Mischvorrichtung einen Kolben zum Austreiben des Mischguts umfasst, der beim Anmischen des Mischguts als Gehäusedeckel dient, wobei der Kolben eine Vielzahl von Federflügeln aufweist, welche an einem Kragen einrastbar sind und welche bei Ausübung einer Kraft in Richtung nach vorne auf den Kolben diesen freigeben, wobei der Kolben mit seinen Federflügeln in Richtung nach vorne bewegbar ist, wobei unter vorne die Richtung verstanden wird, in welche der Kolben zum Ausdrücken des Mischgutes bewegt wird.

Diese Mischvorrichtung umfasst einen Kolben, welcher beim Anmischen als Deckel des Mischraums dient und in welchem eine Stange mit einem Griff zum Bewegen des Mischpaddels geführt ist.

Nach dem Anmischen wird die Stange abgebrochen und mittels einer Applizierpistole kann der Kolben durch erhöhten Kraftaufwand nach vorne bewegt werden und so seine eigentliche Funktion als Kolben zum Auspressen des Knochenzements erfüllen. Es handelt sich bei einer derartigen Vorrichtung um ein leicht bedienbares Einwegsystem.

Um den Kolben in einer ersten Position als Deckel zu sichern sowie um zu ermöglichen, dass der Kolben bei erhöhtem Kraftaufwand aus dieser Position nach vorne gedrückt werden kann, ist zwischen dem Kolben und dem Gehäuse ein Rastring vorgesehen.

### Aufgabe der Erfindung

Der Erfindung liegt demgegenüber die Aufgabe zugrunde, eine eingangs beschriebene Mischvorrichtung für Knochenzement hinsichtlich Montage, Herstellung und/oder Handhabung weiter zu verbessern.

Insbesondere soll ermöglicht werden, einen als Deckel verwendbaren Kolben bereitzustellen, bei welchem hierzu kein zusätzliches Bauteil erforderlich ist.

### Zusammenfassung der Erfindung

Die Aufgabe der Erfindung wird durch eine Mischvorrichtung nach dem unabhängigen Anspruch 1 gelöst.

Bevorzugte Ausführungsformen und Weiterbildungen der Erfindung sind dem Gegenstand der jeweiligen Unteransprüche zu entnehmen.

Die Erfindung betrifft eine Mischvorrichtung, welche insbesondere zum Anmischen von Knochenzement ausgebildet ist.

Die Mischvorrichtung umfasst ein Gehäuse mit einem Mischraum. Der Mischraum ist in der Regel kreiszylindrisch ausgebildet.

Weiter umfasst die Mischvorrichtung einen Kolben zum Austreiben des Mischguts, welcher beim Anmischen des Mischguts als Gehäusedeckel dient.

Vorzugsweise ist in dem Kolben auch eine Stange mit einem Griff zum Bewegen eines Mischpaddels geführt.

Nach dem Anmischen des Mischguts ist der Kolben durch Aufbringen einer Kraft aus der Position als Deckel bewegbar und dient nunmehr dem Austreiben des Mischguts.

Gemäß der Erfindung umfasst der Kolben eine Vielzahl von Federflügeln, welche an einem Kragen des Gehäuses einrastbar sind und welche bei Ausübung einer Kraft auf den Kolben diesen freigeben.

Es hat sich gezeigt, dass es mit direkt am Kolben angeordneten Federflügeln, insbesondere mit Federflügeln, welche einstückig mit dem Kolben bzw. einem Teil des Kolbens ausgebildet sind, möglich ist, einen Einrastmechanismus bereitzustellen, welcher sowohl den Kolben als Deckel in einer ersten Position sicher fixiert als sich auch bei einer definierten höheren Kraft aus dieser Deckelposition herausbewegen und als Kolben verwenden lässt.

Der Kolben wird dabei zusammen mit den Federflügeln nach vorne bewegt. Unter vorne im Sinne der Erfindung wird die Richtung verstanden, in welche der Kolben zum Ausdrücken des Mischgutes bewegt wird.

Hierdurch kann auf weitere Bauteile zum Bereitstellen des Rastmechanismus verzichtet werden, was sowohl Herstellung als auch Montage und Handhabung einfacher und sicherer macht.

Der Kragen ist insbesondere als umlaufende Anlagefläche ausgebildet, welche sich um den gesamten Innenumfang des Gehäuses erstreckt. Vorzugsweise hat der Kragen keine Unterbrechungen. Die Anlagefläche des Kragens erstreckt sich vorzugsweise senkrecht zur Mittelachse des Kolbens.

Vorzugsweise hat der Kolben zumindest 4, besonders bevorzugt zumindest 8, und ganz besonders bevorzugt zumindest 12 Federflügel.

Bei einer bevorzugten Ausführungsform sind in der Position, in welcher der Kolben als Deckel dient, die Federflügel zumindest teilweise unter einem Rasthaken bzw. unter mehreren Rasthaken des Gehäuses verrastet. Gleichzeitig weisen die Federflügel eine gegenüberliegende Auflagefläche auf, mit welcher sie auf einer korrespondierenden Auflagefläche des Kragens des Gehäuses aufliegen.

Der Kragen, welcher der Aufnahme des Kolbens in der Deckelposition dient, bildet somit mit einer Auflagefläche einen vorderen Anschlag. Ein hinterer Anschlag wird durch das Verrasten der Federflügel unter einem Rasthaken des Gehäuses sichergestellt.

Der oder die Rasthaken weisen vorzugsweise oberseitig eine geneigte Gleitfläche auf, um das Einfedern der Federflügel beim Montieren des Kolbens zu erleichtern.

Die Rasthaken sind vorzugsweise integraler Bestandteil des Gehäuses, welches den Mischraum bildet und einstückig mit diesem ausgebildet.

Vorzugsweise nehmen der oder die Rasthaken einen kleineren Umfang ein als die Federflügel, sind somit nur abschnittsweise über den Kragen des Gehäuses verteilt.

So wird auf einfache Weise erreicht, dass der Kolben mit deutlich geringerem Kraftaufwand in der Deckelposition verrastet werden kann, als erforderlich ist, um den Kolben weiter nach vorne zu bewegen.

Die Federflügel sind vorzugsweise L-förmig ausgebildet.

Es ist insbesondere vorgesehen, dass die Federflügel einen Federbereich aufweisen, welcher gegenüber einer Mittelachse des Kolbens geneigt ist, insbesondere in einem Winkel zwischen 10° und 60°. Hierdurch wird verbessert, dass der Kolben auch bei Fertigungstoleranzen mit im Wesentlichen gleichbleibendem Kraftaufwand nach vorne bewegbar ist.

Die Federflügel weisen des Weiteren vorzugsweise einen im Wesentlichen horizontal oder leicht abgeschrägt verlaufenden äußeren Hakenbereich auf. Dieser Hakenbereich liegt in der Deckelposition auf einer Auflagefläche des Kragens auf und ist gleichzeitig hinter zumindest einem Rasthaken verrastet.

Bei einer Weiterbildung der Erfindung umfasst der Kolben einen vorderen Bereich, welcher gegenüber einem dahinterliegenden Bereich einen vergrößerten Durchmesser aufweist. Hierdurch wird bereits ganz vorn am Kolben eine Dichtwirkung erzielt und der Kolben zugleich geführt.

Vorzugsweise befindet sich hinter dem vorderen verdickten Bereich ein weiterer dickerer Bereich, mit welchem der Kolben an der Wand des Mischraums anliegt. Dies kann insbesondere eine Elastomerdichtung, wie beispielweise ein O-Ring sein, welcher in einer Nut des Kolbens eingelassen ist. Durch zumindest zwei axial voneinander beabstandete Auflagebereiche wird so der Kolben sicher geführt.

Die Federflügel befinden sich vorzugsweise, in Bewegungsrichtung des Kolbens gesehen, dahinter.

Bei einer Weiterbildung der Erfindung ist auf das Gehäuse der Mischvorrichtung ein Trichter aufsetzbar welcher, beispielsweise durch eine Trennwand, in zwei Bereiche unterteilt ist.

Ein Bereich kann dem Einfüllen der Flüssigkomponente und ein weiterer Bereich dem Einfüllen der Pulverkomponente dienen.

### Kurzbeschreibung der Zeichnungen

Der Gegenstand der Erfindung soll im Folgenden bezugnehmend auf ein in den Zeichnungen Fig. 1 bis Fig. 11 dargestelltes Ausführungsbeispiel näher erläutert werden.
Fig. 1 zeigt eine perspektivische Ansicht einer Mischvorrichtung.
Fig. 2 zeigt eine Schnittansicht der in Fig. 1 dargestellten Mischvorrichtung.
Fig. 3 zeigt eine Detaildarstellung einer Schnittansicht des Kragenbereiches der Mischvorrichtung.
Fig. 4 zeigt eine schematisierte Schnittdarstellung.
Fig. 5 zeigt eine perspektivische Ansicht von dem Kolben sowie einem im Kolben geführten Mischpaddel mit Griff.
Fig. 6 zeigt eine perspektivische Ansicht des Kolbens.
Fig. 7 zeigt eine perspektivische Detailansicht der Federflügel des Kolbens.
Fig. 8 zeigt eine Detaildarstellung des Kragens des Gehäuses.
Fig. 9 zeigt eine Detaildarstellung des in der Deckelposition verrasteten Kolbens.
Fig. 10 ist eine perspektivische Detaildarstellung der Oberseite der Mischvorrichtung mit aufgesetztem Trichter.
Fig. 11 zeigt eine perspektivische Ansicht der Mischvorrichtung mit aufgeschraubten Applikationsrohr.

### Detaillierte Beschreibung der Zeichnungen

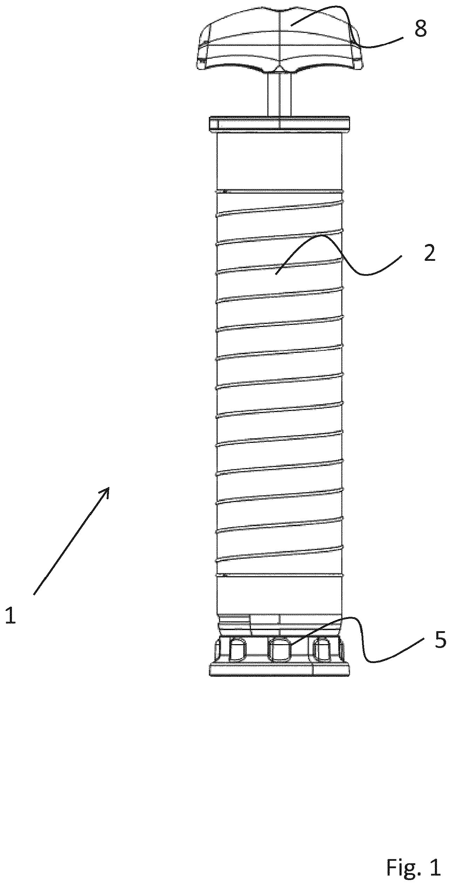

Fig. 1 zeigt in einer perspektivischen Ansicht eine Mischvorrichtung 1.

Die Mischvorrichtung 1 umfasst ein Gehäuse 2. Dieses ist im Wesentlichen kreiszylindrisch angeordnet und dient der Aufnahme des Mischguts.

Das Gehäuse umfasst einen Boden 5, welcher zum Anbringen eines Applikationsrohres abnehmbar, insbesondere abschraubbar ist. Dieses kann beispielsweise nach dem Anmischen des Knochenzements aufgeschraubt werden.

Der Boden 5 ist unten eben ausgebildet und umfasst so eine Standfläche für die Mischvorrichtung 1.

Durch Betätigen eines Mischpaddels über den Griff 8 kann das Mischgut angemischt werden.

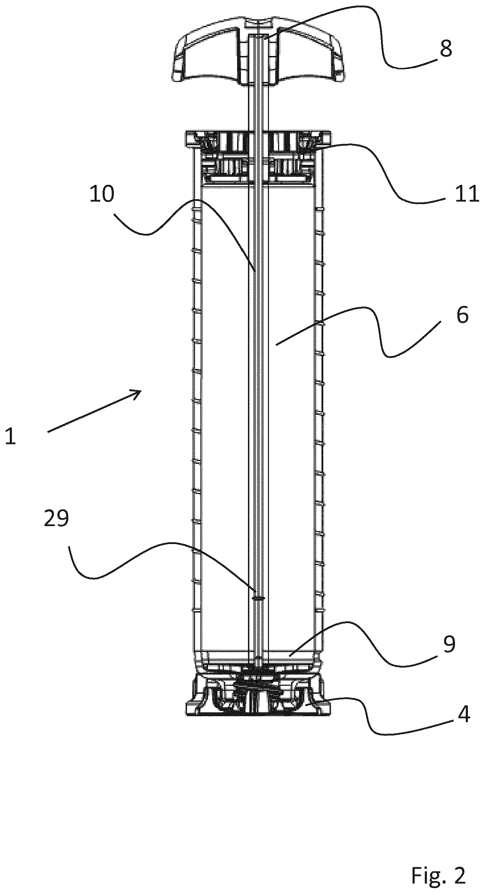

Fig. 2 zeigt eine Schnittdarstellung der in Fig. 1 gezeigten Mischvorrichtung 1. Zu erkennen ist hier der kreiszylindrische Mischraum 6.

Ein Kolben 11, welcher dem Austreiben des Mischguts dient, ist in der hier dargestellten Position vor dem Anmischen des Mischguts als Deckel eingesetzt.

In dem Kolben 11 geführt ist eine Stange 10 zum Antreiben eines Mischpaddels 9. Die Stange 10 und damit das Mischpaddel 9 ist über den Griff 8 bewegbar.

Die Mischvorrichtung 1 kann wie folgt verwendet werden:
Nach dem Einbringen des Mischguts in den Mischraum 6 wird der Kolben 11 zusammen mit dem an der Stange 10 geführten Mischpaddel 9 eingesetzt. Durch Bewegen des Mischpaddels 9 mittels des Griffs 8 wird das Mischgut angemischt.

Sodann wird der Griff 8 nach hinten gezogen.

Der Griff kann zusammen mit der Stange entfernt werden.

Hierzu umfasst die Stange eine Sollbruchstelle 29.

Die Mischvorrichtung 1 kann sodann in eine Applikatorpistole (nicht dargestellt) eingesetzt werden und das Mischgut kann sodann nach Abnahme des Bodens 5 über ein aufgeschraubtes Applikationsrohr 4 ausgetrieben werden, indem der Kolben 11 von der Applikatorpistole nach vorne gedrückt wird.

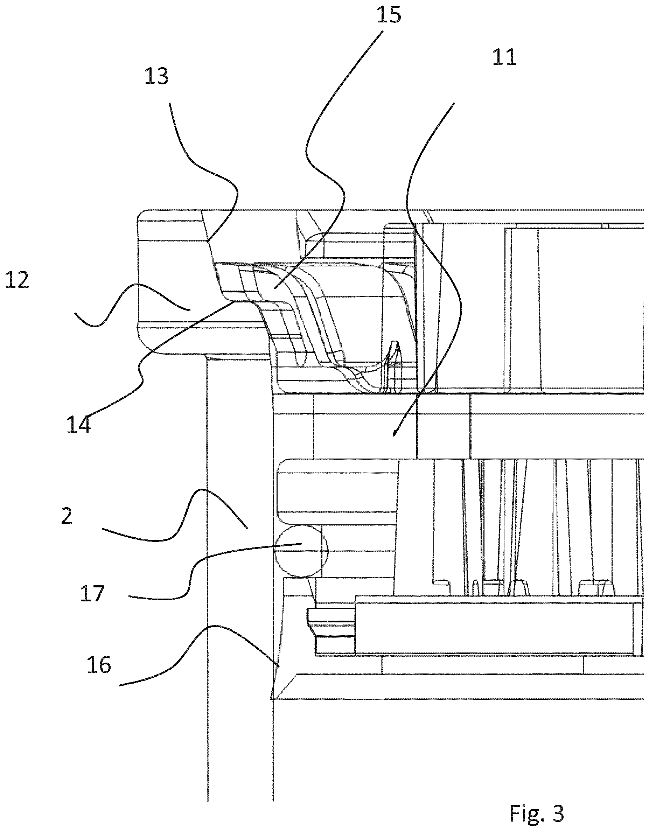

Fig. 3 zeigt eine Detailansicht in einer Schnittdarstellung des Kragenbereiches der Mischvorrichtung.

Zu erkennen ist, dass der Kragen 12 eine geneigte Fläche 13 aufweist.

Der Kolben 11 umfasst im Wesentlichen L-förmig ausgestaltete Federflügel 15.

Hier dargestellt ist die Deckelposition des Kolbens 11.

In dieser Position bildet eine im Wesentlichen horizontal verlaufende Auflagefläche 14 des Kolbens einen Anschlag für die Federflügel 15.

Durch Aufbringen einer erhöhten Kraft federn die L-förmigen Federflügel 15 ein und der Kolben 11 kann nach vorne bewegt werden.

In einem vorderen Bereich 16 vergrößert sich der Durchmesser des Kolbens.

Der vordere Bereich 16 ist als Abstreifer ausgebildet, welcher als separates Bauteil auf den restlichen Kolben aufgesetzt ist, beispielsweise durch Verrasten.

Hinter dem vorderen Bereich 16 ist in einer Nut des Kolbens 11 eine Dichtung, beispielsweise ein O-Ring aus elastomerem Material eingelassen.

Hierdurch wird der Kolben sicher in dem Gehäuse 2 geführt.

Die Federflügel 15 sind einstückig mit dem angrenzenden Kolben 11 ausgebildet. Mithin ist kein separates Bauteil erforderlich, um in den Kolben in der hier dargestellten Deckelposition zu sichern.

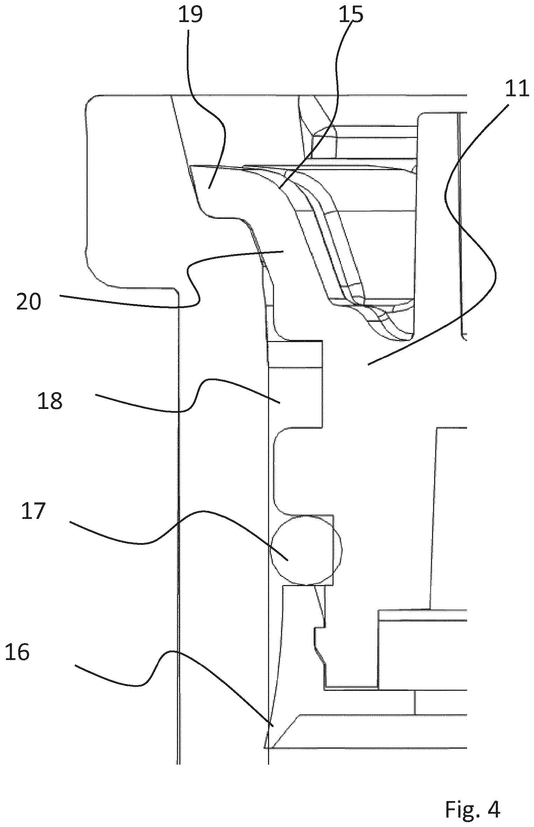

Fig. 4 zeigt eine schematische Schnittdarstellung des Kragenbereiches. Auch hier ist der vordere Bereich 16 des Kolbens 11 mit vergrößertem Durchmesser und die dahinterliegende Dichtung 17 zu erkennen. Bevor der Kolben 11 in die L-förmigen Federflügel 15 übergeht, weist dieser eine umlaufende Nut 18 auf.

Die Federflügel 15 umfassen einen Federbereich 20 sowie einen demgegenüber in einer horizontaleren Richtung verlaufenden Hakenbereich 19.

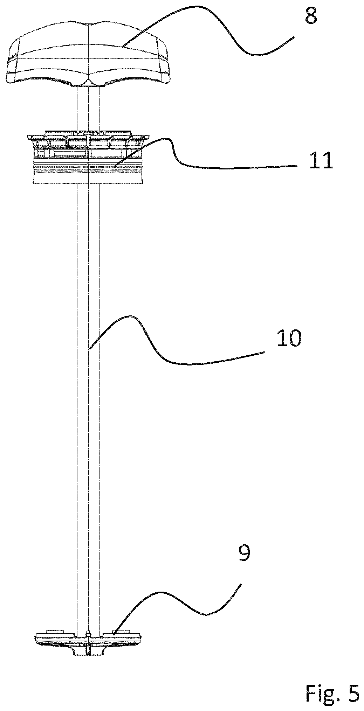

Fig. 5 zeigt eine perspektivische Ansicht des Kolbens 11, in welchem die Stange 10 mit dem Mischpaddel 9 und dem Griff 8 geführt ist.

Diese Bauteile können nach dem Einbringen des Mischguts ggf. noch zusammen mit der Aufnahmeschale (3 in Fig. 1) eingesetzt werden und der Kolben 11 dient als Deckel.

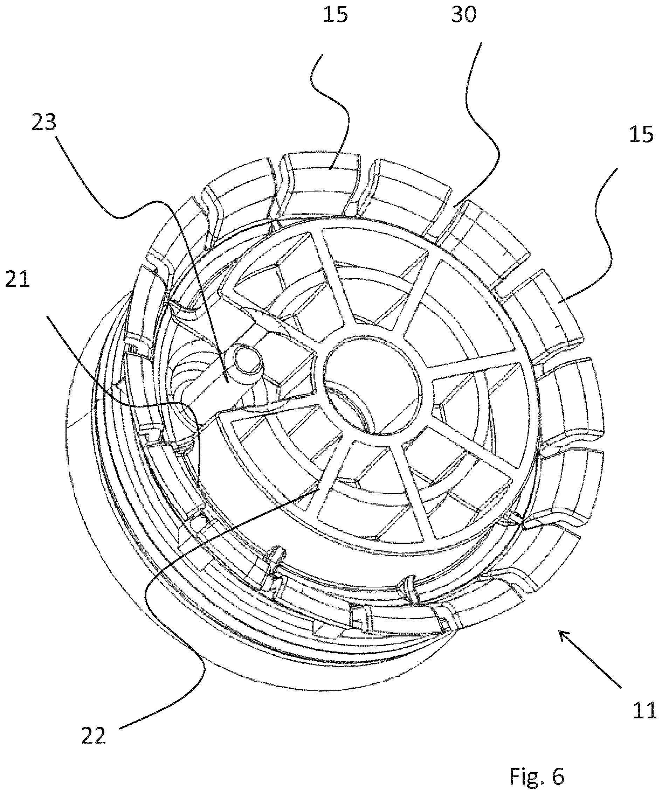

Fig. 6 zeigt eine Detaildarstellung des Kolbens 11. Der Kolben 11 umfasst eine Vielzahl von L-förmigen Federflügeln 15. In diesem Ausführungsbeispiel sind es 16 Federflügel. Vorzugsweise sollte der Kolben zumindest 6 Federflügel haben.

Um einfedern zu können, sind die Federflügel durch Einschnitte 30 voneinander getrennt.

Der Körper des Kolbens 11 wird teilweise durch eine Leichtgewichtsstruktur 22 gebildet, die aus umlaufenden Wänden besteht, welche mittels Stegen verbunden sind.

Weiter umfasst der Kolben 11 auf der Oberseite eine umlaufende Nut 21, welche dem Einsetzen der Aufnahmeschale (3 in Fig. 1) dient.

Zu sehen ist ferner auf der Kolbenoberseite ein Vakuumanschluss 23, mittels dessen die Mischvorrichtung beim Anmischen von Knochenzement an einer Vakuumpumpe angeschlossen wird, um entstehende Gase zu entfernen und Blasenbildung zu vermeiden.

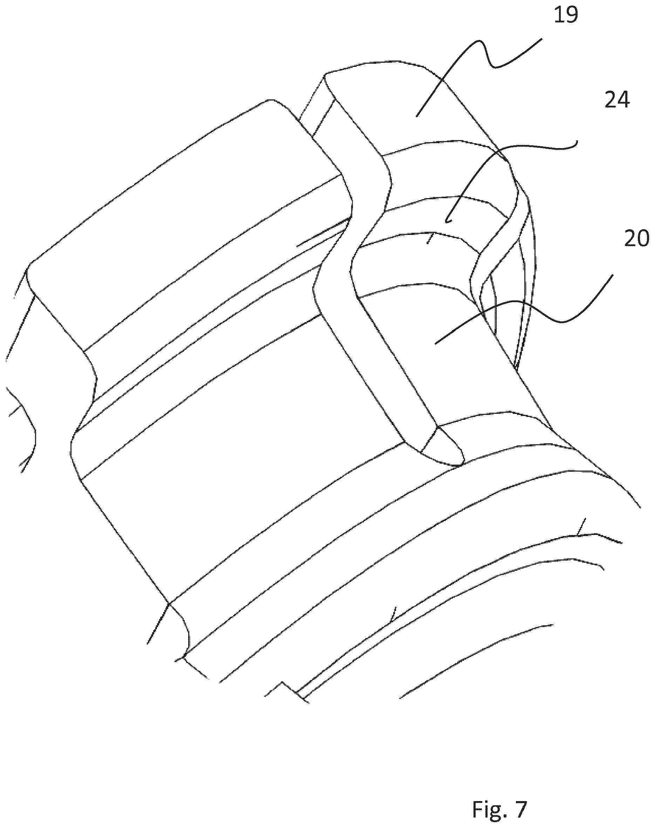

Fig. 7 zeigt eine perspektivische Detaildarstellung der Federflügel des Kolbens.

Zu erkennen ist, dass die Federflügel einen im Wesentlichen vertikal, aber leicht geneigt verlaufenden Federbereich 20 sowie einen sich im Wesentlichen horizontal erstreckenden Hakenbereich 19 aufweisen.

Die Unterseite des Hakenbereiches 19 bildet eine Auflagefläche 24, mittels welcher der Kolben in der Deckelposition auf einer korrespondierenden Auflagefläche des Kragens anliegt (14 in Fig. 3).

Die Übergänge von Hakenbereich 19 und Federbereich 20 sind abgerundet, um ein besseres Gleiten der L-förmigen Federflügel zu ermöglichen.

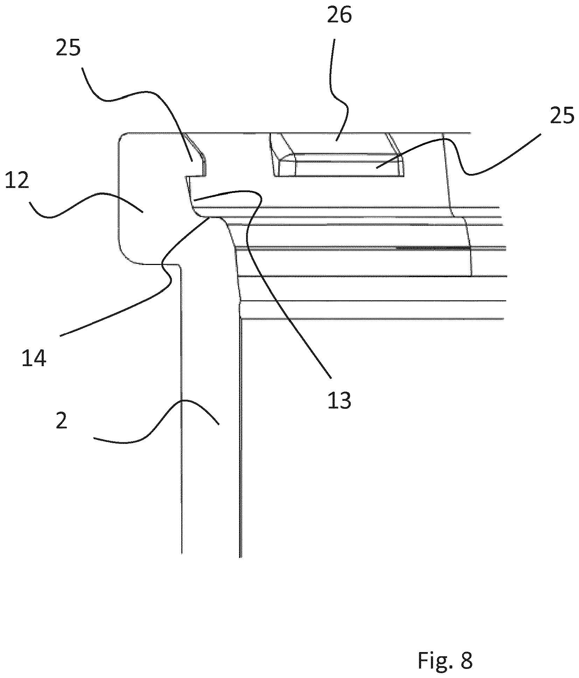

Fig. 8 zeigt eine Detaildarstellung des Kragenbereiches des Gehäuses 2.

Zu erkennen ist, dass der Kragen 12 Rasthaken 25 umfasst. Die Rasthaken 25 umfassen auf der Oberseite eine geneigte Fläche 26, über welche beim Einsetzen des Kolbens die Federflügel gleiten.

Sodann rasten die Federflügel zumindest teilweise oder abschnittweise unter den Rasthaken 25 ein und der Kolben ist gegen ein axiales Nachhintenziehen gesichert. Gleichzeitig liegen die Federflügel vorderseitig an der Anlagefläche 14 an, so dass der Kolben in seiner Deckelposition verrastet ist.

Zu erkennen ist die geneigte Fläche 13, welche ein Einfedern der Federflügel beim Nachvornbewegen des Kolbens erleichtert.

Die Rasthaken 25 nehmen einen geringeren Umfang als die Federflügel ein, um ein Einsetzen des Kolbens mit geringer Kraft zu ermöglichen.

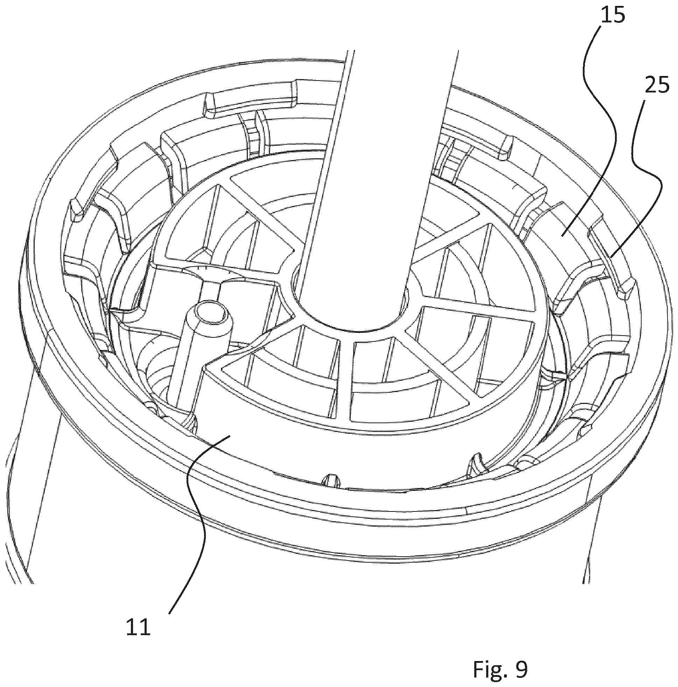

Dies ist besonders gut in Fig. 9 zu erkennen, in welcher eine perspektivische Ansicht des als Deckel verrasteten Kolbens 11 dargestellt ist. Die Federflügel 15 sind abschnittweise unter den Rasthaken 25 verrastet.

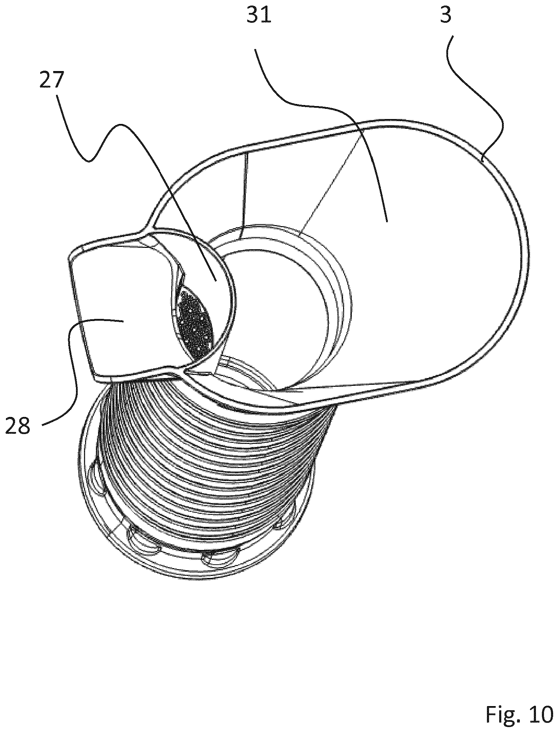

Fig. 10 zeigt eine perspektivische Detaildarstellung des Oberteils der Mischvorrichtung mit aufgesetztem Trichter 3.

Der Trichter 3 wird vor dem Anbringen des Kolbens auf das Gehäuse der Mischvorrichtung gesetzt.

Der Trichter 3 ist durch eine Trennwand 27 in zwei Bereiche unterteilt.

Der größere Bereich 31 des Trichters 3 dient dem Einfüllen der Pulverkomponente.

Der andere Bereich 28 ist rinnenförmig ausgebildet, um das Einfüllen eines Monomers aus einer Ampulle zu erleichtern. Dieser Bereich 28 kann ein Sieb umfassen, um die Gefahr des Eindringens von Glassplittern in den Mischraum zu reduzieren. Der Bereich 31 zum Einfüllen der Pulverkomponente ist dagegen über dem Gehäuse der Mischvorrichtung offen.

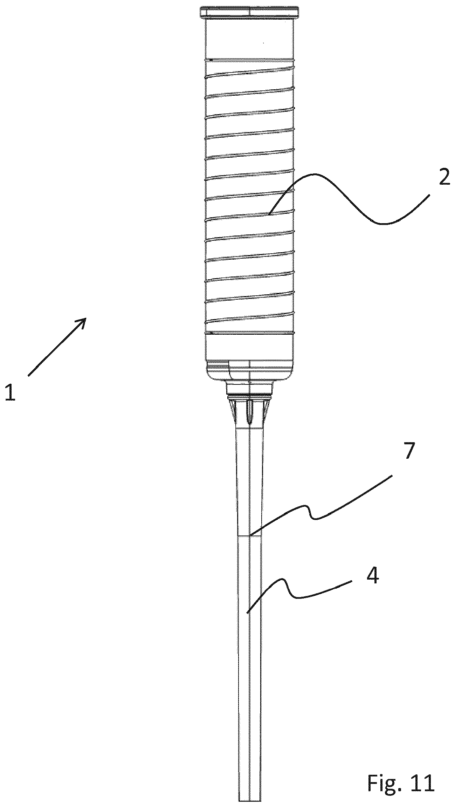

Fig. 11 zeigt die Mischvorrichtung 1 vor dem Auspressen des Knochenzements.

Der Griff (8 in Fig. 1) wurde zusammen mit der Stange (10 in Fig. 2) zum Betätigen des Mischpaddels abgetrennt. Weiter wurde der Boden (5 in Fig. 1) abgeschraubt und stattdessen das Applikationsrohr 4 am Gehäuse 2 angebracht. Vorzugsweise hat das hierfür vorgesehene Gewinde eine hohe Steigung, so dass zum Anbringen weniger als eine Umdrehung erforderlich ist.

Das Applikationsrohr 4 kann eine Sollbruchstelle 7 umfassen, um dieses je nach Anwendung kürzen zu können.

Die hier dargestellte Mischvorrichtung wird in eine Applikatorpistole eingesetzt, mit deren Hilfe das Mischgut ausgetrieben wird.

Durch die Erfindung konnte Handhabung und Montage sowie auch Herstellung einer Mischvorrichtung für Knochenzement vereinfacht werden.

### Bezugszeichenliste

- 1: Mischvorrichtung
- 2: Gehäuse
- 3: Trichter
- 4: Applikationsrohr
- 5: Boden
- 6: Mischraum
- 7: Sollbruchstelle
- 8: Griff
- 9: Mischpaddel
- 10: Stange
- 11: Kolben
- 12: Kragen
- 13: geneigte Fläche
- 14: Auflagefläche
- 15: Federflügel
- 16: vorderer Bereich
- 17: Dichtung
- 18: Nut
- 19: Hakenbereich
- 20: Federbereich
- 21: Nut
- 22: Leichtgewichtsstruktur
- 23: Vakuumanschluss
- 24: Auflagefläche
- 25: Rasthaken
- 26: Gleitfläche
- 27: Trennwand
- 28: rinnenförmiger Bereich
- 29: Sollbruchstelle
- 30: Einschnitt
- 31: Bereich zum Einfüllen der Pulverkomponente

## Patentansprüche

1. Mischvorrichtung (1), insbesondere für Knochenzement, umfassend ein Gehäuse (2) mit einem Mischraum (6), wobei die Mischvorrichtung einen Kolben (11) zum Austreiben des Mischguts umfasst, der beim Anmischen des Mischguts als Gehäusedeckel dient,
wobei der Kolben (11) eine Vielzahl von Federflügeln (15) aufweist, welche an einem Kragen (12) des Gehäuses (2) einrastbar sind und welche bei Ausübung einer Kraft in Richtung nach vorne auf den Kolben (11) diesen freigeben, wobei der Kolben mit seinen Federflügeln in Richtung nach vorne bewegbar ist, wobei unter vorne die Richtung verstanden wird, in welche der Kolben (11) zum Ausdrücken des Mischgutes bewegt wird, und wobei zwischen dem Kolben (11) und dem Gehäuse (2) kein weiteres Bauteil des Rastmechanismus vorhanden ist.

2. Mischvorrichtung nach Anspruch 1, wobei die Federflügel (15) einstückig mit dem Kolben (11) oder einem Teil des Kolbens (11) ausgebildet sind.

3. Mischvorrichtung (1) nach einem der vorstehenden Ansprüche, **dadurch gekennzeichnet, dass** in der Position, in der welcher der Kolben (11) als Deckel dient, die Federflügel (15) zumindest teilweise unter einem Rasthaken (25) des Gehäuses (2) verrastet sind und gleichzeitig mit einer gegenüberliegenden Auflagefläche (24) auf einer korrespondierenden Auflagefläche (14) des Kragens (12) des Gehäuses aufliegen.

4. Mischvorrichtung (1) nach dem vorstehenden Anspruch, **dadurch gekennzeichnet, dass** der Rasthaken (25) oberseitig eine geneigte Gleitfläche (26) aufweist.

5. Mischvorrichtung (1) nach einem der vorstehenden Ansprüche, **dadurch gekennzeichnet, dass** in dem Kolben (11) eine Stange (10) zum Bewegen eines Mischpaddels (9) geführt ist.

6. Mischvorrichtung (1) nach einem der vorstehenden Ansprüche, **dadurch gekennzeichnet, dass** die Federflügel (15) L-förmig ausgebildet sind.

7. Mischvorrichtung (1) nach einem der vorstehenden Ansprüche, **dadurch gekennzeichnet, dass** die Federflügel (15) einen Federbereich (20) aufweisen, welcher gegenüber eine Mittelachse des Kolbens (11) geneigt ist, insbesondere in einem Winkel zwischen 10° und 60°.

8. Mischvorrichtung (1) nach einem der vorstehenden Ansprüche, **dadurch gekennzeichnet, dass** der Kolben einen vorderen Bereich (16) aufweist, welcher gegenüber einem dahinter liegenden Bereich einen vergrößerten Durchmesser aufweist.

9. Mischvorrichtung (1) nach einem der vorstehenden Ansprüche, **dadurch gekennzeichnet, dass** die Mischvorrichtung (1) einen auf das Gehäuse (2) aufsetzbaren Trichter (3) umfasst, welcher in zwei Bereiche (28, 31) unterteilt ist.

10. Mischvorrichtung (1) nach dem vorstehenden Anspruch, **dadurch gekennzeichnet, dass** die Mischvorrichtung einen abnehmbaren, insbesondere abschraubbaren, Boden (5) umfasst, welcher gegen ein Applikationsrohr (4) austauschbar ist.

## Claims

1. A mixing device (1) in particular for bone cement, comprising a housing (2) with a mixing cavity (6), the mixing device comprising a plunger (11) for forcing out mixed material, the plunger being used as a housing cover during mixing of the material to be mixed;
wherein the plunger (11) has a plurality of resilient tabs (15) which can be latched on a collar (12) of the housing (2) and which release the plunger (11) when a force is applied to the plunger in a forward direction, wherein the plunger can be moved in said forward direction together with its resilient tabs, forward referring to the direction in which the plunger (11) is moved to force out the mixed material, and wherein there is no further component of the latching mechanism between the plunger (11) and the housing (2).

2. The mixing device according to claim 1, wherein the resilient tabs (15) are formed integrally with the plunger (11) or with a portion of the plunger (11).

3. The mixing device (1) according to any one of the preceding claims, **characterised in that** in a position in the which the plunger (11) serves as a cover, the resilient tabs (15) are at least partially latched under a locking hook (25) of the housing (2) while an opposite engagement surface (24) thereof rests on a corresponding bearing surface (14) of the collar (12) of the housing.

4. The mixing device (1) according to the preceding claim, **characterised in that** the locking hook (25) has an inclined sliding surface (26) on its upper side.

5. The mixing device (1) according to any one of the preceding claims, **characterised in that** a rod (10) for moving a mixing paddle (9) is guided inside the plunger (11).

6. The mixing device (1) according to any one of the preceding claims, **characterised in that** the resilient tabs (15) are L-shaped.

7. The mixing device (1) according to any one of the preceding claims, **characterised in that** the resilient tabs (15) have a resilient portion (20) which is inclined relative to a central axis of the plunger (11), in particular at an angle between 10° and 60°.

8. The mixing device (1) according to any one of the preceding claims, **characterised in that** the plunger has a front portion (16) which has an enlarged diameter compared to a rearward portion.

9. The mixing device (1) according to any one of the preceding claims, **characterised in that** the mixing device (1) comprises a funnel (3) that can be placed on the housing (2) and which is divided into two portions (28, 31).

10. The mixing device (1) according to the preceding claim, **characterised in that** the mixing device comprises a detachable, in particular unscrewable base (5) which can be replaced by an application tube (4).

## Revendications

1. Dispositif de mélangeage (1), en particulier pour ciment osseux, incluant un boîtier (2) avec une chambre de mélangeage (6), le dispositif de mélangeage incluant pour expulser le produit mélangé un piston (11) qui, lors du mélangeage du produit mélangé, sert de couvercle de boîtier, le piston (11) comportant une pluralité d'ailettes élastiques (15) qui sont encliquetables sur un collet (12) du boîtier (2) et qui en exerçant sur le piston (11) une force en direction de l'avant libèrent celui-ci, le piston étant déplaçable avec ses ailettes élastiques en direction de l'avant, par avant étant entendue la direction dans laquelle le piston (11) est déplacé pour faire sortir le produit mélangé, et aucun autre composant du mécanisme d'encliquetage n'étant présent entre le piston (11) et le boîtier (2).

2. Dispositif de mélangeage selon la revendication 1, les ailettes élastiques (15) étant conçues d'un seul tenant avec le piston (11) ou avec une partie du piston (11),

3. Dispositif de mélangeage (1) selon une des revendications précédentes, **caractérisé en ce que**, dans la position dans laquelle le piston (11) sert de couvercle, les ailettes élastiques (15) sont encliquetées au moins en partie sous un crochet d'encliquetage (25) du boîtier (2) et, en même temps, prennent appui par une surface d'appui opposée (24) sur une surface d'appui correspondante (14) du collet (12) du boîtier.

4. Dispositif de mélangeage (1) selon la revendication précédente, **caractérisé en ce que** le crochet d'encliquetage (25) comporte dans sa partie supérieure une surface de glissement inclinée (26).

5. Dispositif de mélangeage (1) selon une des revendications précédentes, **caractérisé en ce que** dans le piston (11) est guidée une tige (10) pour déplacer une palette de mélangeage (9).

6. Dispositif de mélangeage (1) selon une des revendications précédentes, **caractérisé en ce que** les ailettes élastiques (15) sont conçues en forme de L.

7. Dispositif de mélangeage (1) selon une des revendications précédentes, **caractérisé en ce que** les ailettes élastiques (15) comportent une zone élastique (20) qui est inclinée par rapport à un axe médian (11), en particulier selon un angle entre 10° et 60°,

8. Dispositif de mélangeage (1) selon une des revendications précédentes, **caractérisé en ce que** le piston comporte une zone avant (16) qui présente un diamètre agrandi par rapport à une zone située derrière elle.

9. Dispositif de mélangeage (1) selon une des revendications précédentes, **caractérisé en ce que** le dispositif de mélangeage (1) inclut un entonnoir (3) qui peut être monté sur le boîtier (2) et qui est divisé en deux zones (28,31).

10. Dispositif de mélangeage (1) selon la revendication précédente, **caractérisé en ce que** le dispositif de mélangeage inclut un fond (5) amovible, en particulier dévissable, qui est remplaçable par un tube applicateur (4).
